# EUROPEAN PATENT APPLICATION

(11) **EP 2 001 188 A1**
(43) Date of publication of application: **10.12.2008**
(21) Application number: 07011313.9
(22) Date of filing: 08.06.2007
(51) Int. Cl.: H04L 29/06, G06F 19/00

(54) **Method for authenticating a medical device and a remote device**

(71) Applicant: F.HOFFMANN-LA ROCHE AG, 4070 Basel (CH); Roche Diagnostics GmbH, 68305 Mannheim (DE)
(72) Inventor: Ehrsam, Matthias, CH-3065 Bolligen (CH); Frikart, Marcel, CH-3012 Bern (CH); Jungen, Markus, CH-3065 Bolligen (CH); Oberli, Markus, CH-3422 Kirchberg (CH); Frey, Christian, CH-5013 Niedergösgen (CH); Frikart, Philippe, CH-3503 Gysenstein (CH)
(74) Representative: Fischer, Britta Ruth

(57) **Abstract**

The invention relates to a method for authenticating a medical device (1), in particular an infusion pump, and a remote device (2), wherein the medical device (1) and the remote device (2) are placed in communication range and an authentication phase is performed, the authentication phase comprising the steps of generating and displaying a PIN code by the medical device (1), waiting for a user input of the displayed PIN code into the remote device (2), checking the authentication of the entered PIN code by the medical device (1), I generating a strong key by the medical device (2), sending the strong key encrypted to the remote device (2), checking the authentication of the sent strong key by the medical device (1) and upon successful authentication storing the strong key in a non-volatile memory of the medical device (1) and of the remote device (2). Preferably, a pairing phase is performed before the authentication phase.

## Description

The invention relates to a method for authenticating a medical device and a remote device according to the preamble of claim 1. The method preferably comprises a pairing phase and an authentication phase.

A medical device and a remote device authenticated by the method may for example be an infusion pump and a diabetes management device, wherein the infusion pump is the medical device and the diabetes management device is the remote device or vice versa. The remote device may also be, for example, a communication device, a blood glucose meter, a smart phone, a personal digital assistant (PDA), or a remote terminal representing a remote infusion pump display on which data from the infusion pump is displayed to a user. The remote device may also include a bolus advisor by which the user can command the administration of insulin through the infusion pump and on which the delivery history of the infusion pump is displayed. Other types of medical devices and remote devices are possible. The functionality of the medical device and the remote device may also be reversed, i.e. the medical device may be one of the above devices mentioned as examples for a remote device and the remote device may be one of the above devices mentioned as examples for a medical device.

It is an object of the claimed invention to provide a method for ensuring a reliable, safe and secure communication between a medical device and a remote device. The method shall be initiated before the first connection between a medical device and a remote device in order to enable a secure, reliable and safe communication between the medical device and the remote device. Furthermore, the method shall be performed upon replacement of either the medical device or the remote device.

In order to implement these and still further objects of the invention, which will become more readily apparent as the description proceeds, a method for authenticating a medical device, in particular an infusion pump, and a remote device is provided, which comprises the steps of placing the medical device and the remote device in communication range and performing an authentication phase. The authentication phase comprises the steps of generating and displaying a PIN code by the medical device, waiting for a user input of the displayed PIN code into the remote device, checking the authentication of the entered PIN code by the medical device, generating a strong key by the medical device, sending the strong key encrypted to the remote device, checking the authentication of the sent strong key by the medical device and upon successful authentication storing the strong key in a non-volatile memory of the medical device and in a non-volatile memory of the remote device. Hereby authentication is understood as the act of verifying the identity of a device. Authentication is a way to ensure that a device which attempts to perform functions in connection with another device is in fact the device which is authorised to do so.

The PIN code is preferably a 10-digits numeric or alphanumeric value, whereas the strong key is preferably a 128-bit value.

For encrypting the strong key preferably symmetric cryptography is used, employing the same key for encrypting and decrypting. For example a cryptography method based on AES (Advanced Encryption Standard), DES (Data Encryption Standard), IDEA (International Data Encryption Algorithm), Blowfish, or similar may be used.

According to a preferred embodiment of the invention a pairing phase is performed before performing the authentication phase. Preferably Bluetooth is used as communication protocol for the pairing phase if wireless communication is used. Of course other communication protocols for wireless, radio frequency or wired communication may be used. In the pairing phase the remote device is set into a pairable mode in order to be discoverable by the medical device. The medical device is set in pairing mode and a remote device discovery inquiry is performed by the medical device, i.e. the medical device searches for remote devices to pair with. Then the discovered remote devices are displayed for user selection. Upon user selection of a remote device the medical device and the selected remote device pair with one another. Upon successful pairing a link key is generated on the medical device and on the remote device and this link key and the virtual address of the remote device are stored in a non-volatile memory in the medical device, while the link key and the virtual address of the medical device are stored in a non-volatile memory in the remote device. Then the medical device enters connectable mode and the remote device is connected to the medical device. After that the authentication phase is performed.

Pairing the remote device and the medical device means to establish a trusted relationship by learning a shared secret represented by the link key. As the medical device knows the virtual address of the remote device, the medical device only accepts requests from a remote device with this particular virtual address and hence responds to direct connection requests from this remote device.

When performing the remote device discovery inquiry the discovered remote devices are preferentially filtered so that only remote devices that are in the pairable mode are displayed for user selection. Furthermore, the discovered remote devices may be filtered prior to displaying for user selection by comparing the discovered remote devices to entries of a pre-defined remote device list, the pre-defined remote device list being preferably stored in the medical device.

The pairing of the medical device and the remote device is preferably carried out by requesting authentication of the remote device, which has been selected by the user, by the medical device and vice versa, whereby for the authentication the medical device and the selected remote device each provide a fixed PIN code.

With the method according to the invention a medical device and the remote device can be mutually authenticated to ensure reliable, safe and secure connection and communication between the medical device and the remote device, whereby an authentication phase of the method is preferably preceded by a pairing phase. The method according to the invention can be applied for a first or initial connection between a medical device and a remote device (so called out-of-the-box connection). Furthermore, the method according to the invention can be applied when the medical device and/or the remote device have been replaced. The method according to the invention is easy to use and user-friendly and can be accomplished, for example, by a patient with diabetes, a health care professional, a nurse, a sales representative, a training expert or any other trustworthy person.

To increase safety, it is preferred that at maximum one remote device can be connected to the medical device simultaneously, whereas only trusted remote devices may be connected to the medical device to remotely control and/or access the medical device upon performance of the method for authentication according to the invention. Connecting a remote device to the medical device prevents the medical device to be connectable to another remote device until it disconnects from the remote device. To further ensure a safe connection, preferably a remote device can be connected with maximum one medical device. Authentication of the devices is required to ensure safety. User identification is, however, not required.

The pairing phase according to the preferred embodiment of the method according to the invention may preferably only be initiated through direct user interaction to further increase safety. Hence, it shall not be possible to initiate the pairing phase by radio frequency. By combining the authentication phase with a pairing phase as in the preferred embodiment of the method according to the invention overall safety is further improved.

Assuming that the medical device offers several services to the remote device, the authentication achieved by applying the method according to the invention shall be valid for all of these services, i.e. the medical device and the remote device are actually authenticated and it is superfluous to perform the authentication again separately for each service offered by the medical device.

Further advantageous features and applications of the invention can be found in the dependent claims as well as in the following description of the drawings illustrating the invention. In the drawings like reference signs designate the same or similar parts throughout the several figures of which:
Figure 1 shows an abstract interconnection layer model of the medical device and the remote device,
Figure 2 shows a schematic representation of a first part of the method according to the invention which comprises the pairing phase, and
Figure 3 shows a schematic representation of a second part of the method according to the invention which comprises the authentication phase.

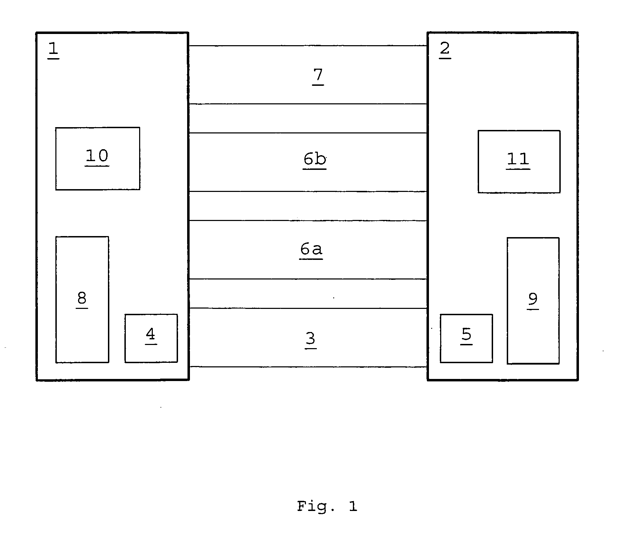

Figure 1 shows a medical device 1 and a remote device 2 which shall be authenticated by the method according to the invention before a secure link for data transmission is established between the two devices 1, 2. The medical device 1 may for example be an infusion pump and the remote device 2 may for example be a diabetes management system.

The method according to the invention comprises an authentication phase and preferably also a pairing phase, which is executed before the authentication phase. For the pairing phase, in which the medical device 1 and the remote device 2 shall be paired, i.e. a trusted relationship between the medical device 1 and the remote device 2 shall be established, a Bluetooth layer 3 is provided through which a Bluetooth module 4 of the medical device 1 and a Bluetooth module 5 of the remote device 2 may communicate. Of course, another protocol for wireless and wired networks may be used instead of the Bluetooth protocol.

After successful pairing of the medical device 1 and the remote device 2, the medical device 1 and the remote device 2 are connected via the transport layer 6a. With the transport layer 6a is associated a security layer 6b. Physically the transport layer 6a and the security layer 6b may form one single layer. After successful authentication of the medical device 1 and the remote device 2, the transport layer 6a is basically used for error recognition and retransmission of data.

To increase communication security and to ensure true end-to-end communication security an authentication phase is performed after the pairing phase, whereby the authentication is performed via the security layer 6b. After successful performance of the authentication phase, a secure link is established between the medical device 1 and the remote device 2 and commands can be sent and data can be transmitted between the medical device 1 and the remote device 2 via an application layer 7.

The medical device 1 and the remote device 2 each comprise a control unit 8, 9, preferably in form of a microprocessor, that is associated with the transport layer 6a, the security layer 6b and the application layer 7. Furthermore, the medical device 1 and the remote device 2 each comprise a user interface 10, 11, associated with the control units or microprocessors 8, 9, respectively. The user interfaces 10, 11 of the medical device 1 and the remote device 2 each preferably comprise a display (not shown).

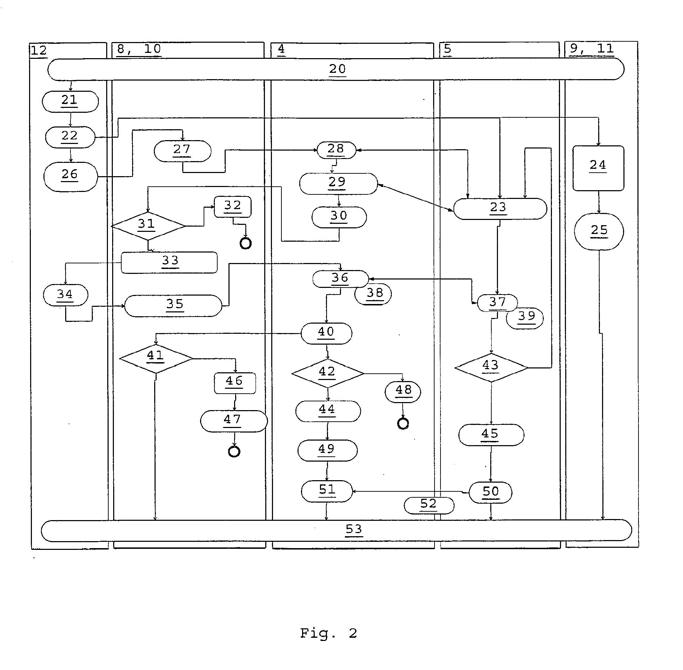

Figure 2 shows a schematic representation in form of a flowchart of a first part of the method according to the invention. The steps/items of the flowchart are assigned to the person or units who actually perform the steps. Hence, the steps/items are assigned to the user 12, the microprocessor 8 of the medical device 1, the Bluetooth module 4 of the medical device 1, the Bluetooth module 5 of the remote device 2 or the microprocessor 9 and the user interface 11 of the remote device 2.

A precondition for the method according to the invention is that on the side of the medical device 1 no remote device 2 is paired. Step 20 denotes the start of the method according to the invention. In step 21 the medical device 1 and the remote device 2 are placed in range by a user 12 for communication and connection. The medical device 1 and the remote device 2 are preferably placed in a safe and private environment.

Hereafter, the pairing phase of the method according to the invention is initiated, the pairing phase being optional. In step 22 the remote device 2 is set into pairable mode. For this the user 12 preferably presses a specific key on the remote device 2 on powerup. Pressing this specific key activates a specific pairing firmware on the Bluetooth module 5 as well as on the microprocessor 9 of the remote device 2. The Bluetooth module 5 then sets the remote device 2 in step 23 in pairable mode, i.e. in the mode of being Bluetooth inquirable and connectable, in order to be Bluetooth discoverable by the medical device 1. The user interface 11 of the remote device 2 preferably shows the message "Starting pairing and authentication process" or a similar message on its display (step 24) and the microprocessor 9 of the remote device 2 tries to communicate with the medical device 1 through the transport layer 6a (step 25; confer Figure 1). Communication via the transport layer 6a will, however, first be possible upon successful completion of the pairing phase, i.e. upon successful pairing of the Bluetooth modules 4, 5 of the medical device 1 and the remote device 2.

In step 26 the medical device 1 is set into pairing mode, preferably by the user 12 selecting a corresponding pairing menu which is displayed on the user interface 10 of the medical device 1. The microprocessor 8 of the medical device 1 then starts the actual Bluetooth pairing process in step 27 by prompting the Bluetooth module 4 of the medical device 1 to initiate and perform a remote device discovery inquiry in step 28. During the remote device discovery inquiry the Bluetooth module 4 searches for potential pairable remote devices 2 in range, thereby basically obtaining the virtual addresses of the Bluetooth modules 5 of the remote devices 2 in range. In step 29 the discovered remote devices 2 are preferably filtered so that only pairable remote devices 2, i.e. remote devices 2 in the pairable mode, are found. Hence, mobile phones or similar electronic devices, which are not aimed at being connected with the medical device 1, can be filtered out. In step 30 the Bluetooth module 4 sends the found remote devices 2 to the microprocessor 8 of the medical device 2.

The microprocessor 8 of the medical device 2 checks in step 31 if any remote devices 2 have been found during the remote device discovery inquiry. If no remote devices 2 have been found then the user interface 10 of the remote device 2 preferably displays in step 32 the message "No remote devices found" or a similar message and the method according to the invention is ended. If remote devices 2 have been found during the remote device discovery inquiry then the discovered, pairable remote devices 2 are displayed as a list on the user interface 10 of the medical device 1 (step 33). Preferably the Bluetooth friendly names of the found remote devices 2 are displayed, wherein the Bluetooth friendly names preferentially contain parts of the serial numbers of the remote devices 2.

In step 34 the user 12 selects a remote device 2 from the list of discovered remote devices 2 that is displayed on the user interface 10 of the medical device 2. The microprocessor 8 of the medical device 1 then sends the selected remote device 2, i.e. the information which remote device 2 has been selected, to the Bluetooth module 4 (step 35) and prompts the Bluetooth module 4 to start with the Bluetooth pairing. In step 36 the Bluetooth module 4 initiates the Bluetooth pairing with the selected remote device 2 by requesting authentication (step 37 for the Bluetooth module 5 of the remote device 2).

The pairing is performed on the Bluetooth layer 3 (confer Figure 1) and the Bluetooth modules 4, 5 of the medical device 1 and of the remote device 2 are both prompted for authentication to provide a PIN code, which can be hard-coded on each of the Bluetooth modules 4, 5, i.e. it is a fixed PIN code (steps 38, 39). In step 40 the Bluetooth module 4 of the medical device 1 sends the pairing result to the microprocessor 8 of the medical device 1, the pairing results comprising the authenticated identity of the remote device 2. In steps 41, 42, 43 the microprocessor 8, the Bluetooth module 4 of the medical device 1 and the Bluetooth module 5 of the remote device 2 each check if the pairing has been successful, i.e. if the identity of the other device 2, 3 has been authenticated successfully. On successful authentication, i.e. on successful pairing, the Bluetooth modules 4, 5 of the medical device 1 and of the remote device 2 each generate a unique link key and store this link key and the Bluetooth address of the other device 2, 1 locally in a non-volatile memory of the medical device 1 and of the remote device 2, respectively (steps 44 and 45). This has the advantage that the pairing has not to be re-done before each establishment of a connection between the medical device 1 and the remote device 2.

If the pairing has not being successful, then the medical device 1 shows on its display 10 preferably a corresponding message, for example "Process not successful" (step 46). The microprocessor 8 of the medical device 1 then puts the Bluetooth module 4 of the medical device 1 into idle state (step 48), exits the pairing menu, which is preferably displayed on the user interface 10 of the medical device 1 and powers off the Bluetooth module 4 (step 47). The Bluetooth module 5 of the remote device 2 sets the remote device 2 back into pairable mode, i.e. the method returns to step 23, in order to be again Bluetooth discoverable by the medical device 1.

Upon successful pairing and link key storage the Bluetooth module 4 of the medical device 1 makes itself Bluetooth connectable via the Bluetooth layer 3 (step 49; confer Fig. 1). Then in step 50 the Bluetooth module 5 of the remote device 2 achieves to establish a Bluetooth connection with the medical device 1. A Bluetooth connection via a Bluetooth serial port profile or any other appropriate profile is then actually established in step 51. The medical device 1 and the remote device are then Bluetooth connected (item 52).

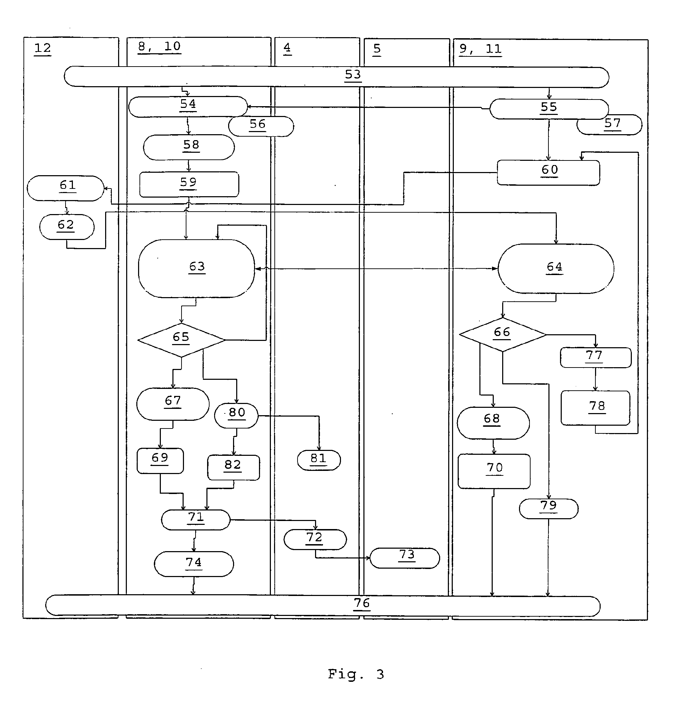

The pairing of the medical device 1 and the remote device 2 is now completed, in that communication between the two devices 1, 2 is possible via the Bluetooth layer 3 (Fig. 1). The method according to the invention is then continued in step 53 with the authentication phase, the authentication phase being depicted in Figure 3 as flowchart.

Figure 3 shows a schematic representation in form of a flowchart of the authentication phase of the method according to the inventions. As for Figure 2 the steps/items of the flowchart are assigned to the user 12, the microprocessor 8 of the medical device 1, the Bluetooth module 4 of the medical device 1, the Bluetooth module 5 of the remote device 2 or the microprocessor 9 and the user interface 11 of the remote device 2. Item 53 represents the completed pairing phase (confer Figure 2). After completion of the pairing phase the microprocessor 9 of the remote device 2 is able to establish a transport layer communication via the transport layer 6a (confer Figure 1) with the microprocessor 8 of the medical device 1 (steps 54 and 55). Then, the medical device 1 and the remote device 2 are connected via the transport layer 6a (items 56 and 57). In step 58 the microprocessor 8 of the medical device 1 generates a numeric PIN code in form of 10 digit number and shows this PIN code in step 59 on the display of the user interface 10 of the medical device 1. The microprocessor 8 of the medical device 1 then waits for the authentication of the remote device 2.

The microprocessor 11 of the remote device 2 asks the user 12 in step 60 via the user interface 11 of the remote device 2 to enter the PIN code that is currently displayed on the user interface 10 of the medical device 1. In step 61 the user 12 reads the PIN code displayed on the display of the user interface 10 of the medical device 1 and enters in step 62 the PIN code into the user interface of the remote device 2.

In steps 63 and 64 the microprocessor 8 of the medical device 1 checks then authentication of the PIN code which has been entered by the user 12 into the remote device 2. For authentication the microprocessor 8 of the medical device 1 generates a signature from the PIN code. The microprocessor 9 of the remote device 2 also generates a signature from the entered PIN code, for example by applying a symmetric encryption method such as AES, DES, IDEA, Blowfish or similar, and attaches the signature to a data packet, which it sends to the microprocessor 8 of the medical device 1. The microprocessor 8 of the medical device 1 then compares the signature which is attached to the sent data packet to the signature it has generated itself. If the signatures are the same, then authentication has been successful. Other authentication methods can be applied alternatively or additionally.

After authenticating the PIN code the microprocessor 8 of the medical device 1 generates a strong key in form of a 128 bit number and sends the strong key encrypted to the remote device 2. The remote device 2 may also request sending of the strong key from the medical device 1. The above-mentioned encryption methods can be used. Then the microprocessor 8 checks authentication of the strong key that has been received by the microprocessor 9 of the remote device 2, wherein authentication may be performed as described for the PIN code. After authentication of the strong key the medical device 1 and the remote device 2, i.e. their microprocessors 8, 9, preferably exchange their serial numbers, which then can be displayed to the user 12 via the user interfaces 10, 11.

Each microprocessor 8, 9 of the medical device 1 and the remote device 2 checks in steps 65 and 66 if the authentication performed in steps 63 and 64 has been successful. On successful authentication the medical device 1 and the remote device 2 each store the strong key in a non-volatile memory (steps 67 and 68). Furthermore, the medical device 1 and the remote device 2 preferably each inform the user 12 via their user interfaces 10, 11 with the message "Process successful" or a similar message about the successful authentication (steps 69 and 70). In step 70 the remote device 2 preferentially additionally prompts the user 12 to restart the remote device 2. In step 71 the microprocessor 8 of the medical device 1 preferably commands the Bluetooth module 4 to disconnect, leading to a disconnection of the Bluetooth modules 4, 5 in items 72, 73. In step 74 the medical device 1 then preferably exits the pairing menu and shuts down the Bluetooth module 4.

The method according to the invention is now completed successfully as indicated by step 76 and the medical device 1 and the remote device 2 are authenticated for future use and communication and connection via the application layer 7 (confer Figure 1) after start-up. For future communication the strong key is used, which is stored on the medical device 1 and on the remote device 2.

If authentication has not been successful the microprocessor 8 of the medical device 1 returns to step 63, i.e. it returns to checking the authentication of the PIN code entered by the user 12 into the remote device 2. The remote device 2 preferentially shows a corresponding message, such as "Wrong PIN code", on the display of its user interface 11 (step 77). Furthermore, it may show the last entered PIN code on its display (step 78), so that the user may compare the last entered PIN code with the PIN code actually shown on the display of the medical device 1 (step 59). Upon informing the user 12, that authentication was not successful, the microprocessor 9 of the remote device 2 returns to step 60 and waits for the user 12 to enter the PIN code which is actually shown on the display of the medical device 1. If the remote device displays the last entered PIN code on its display, then the user 12 may correct the PIN code entry manually to the PIN code shown on the display of the medical device 1.

If authentication has not been successful because a time-out occurred during authentication or because the user 12 has performed an exit on the medical device 1 or has powered off the remote device 2 during authentication, then the microprocessor 8 of the medical device 1 commands in step 80 the Bluetooth module 4 of the medical device 1 to unpair the medical device 1 and the remote device 2. Thereupon the Bluetooth module 4 of the medical device 1 discards its pairing data, i.e. the link key stored during the pairing phase, and such un-pairs the medical device 1 and the remote device 2 in step 81. Preferably the medical device 1 displays a corresponding message, such as "Process not successful" for the user 12 (step 82). Then the Bluetooth connection is disconnected in steps 71, 72 and 73 as described above. Finally the microprocessor 8 of the medical device 8 exits a possible pairing menu and shuts down the Bluetooth module 4 of the medical device 1 (step 74). The microprocessor 9 of the remote device 2 then shuts down the remote device 2 in step 79 and the method according to the invention has to be repeated for successful authentication.

An existing pairing and authentication between a medical device and a remote device can be repealed by deleting the link key and the virtual address as well as the strong key stored in a non-volatile memory of the medical device and in a non-volatile memory of the remote device on either the medical device or the remote device or on both the devices. This can simply be initiated by the user by selecting a corresponding un-pairing menu which is displayed on the user interface of the medical device or by pressing a specific key on the remote device on power up.

In another embodiment of the invention, an existing pairing and authentication between a medical device and a remote device can be repealed by starting a new pairing and authentication process or sequence, respectively, on either the medical device or the remote device. Thereby, the link key and the virtual address as well as the strong key stored in a non-volatile memory of the medical device and in a non-volatile memory of the remote device are deleted on that device on which the pairing and authentication process/sequence has been started prior to the pairing and authentication process being performed or will be overwritten during the performance of the pairing and authentication process/sequence.

## Claims

1. A method for authenticating a medical device (1), in particular an infusion pump, and a remote device (2), **characterised in that** the medical device (1) and the remote device (2) are placed in communication range and an authentication phase is performed, the authentication phase comprising the steps of
- generating and displaying a PIN code by the medical device (1),
- waiting for a user input of the displayed PIN code into the remote device (2),
- checking the authentication of the entered PIN code by the medical device (1),
- generating a strong key by the medical device (2),
- sending the strong key encrypted to the remote device (2),
- checking the authentication of the sent strong key by the medical device (1) and
- upon successful authentication storing the strong key in a non-volatile memory of the medical device (1) and of the remote device (2).

2. The method according to claim 1, wherein after authentication of the strong key the medical device (1) and the remote device (2) exchange their serial numbers.

3. The method according to claim 1 or 2, wherein upon successful authentication the remote device (2) prompts the user (12) to restart the remote device (2).

4. The method according to one of the preceding claims, wherein upon non-successful authentication the method returns to the step of waiting for a user input of the displayed PIN code into the remote device (2), preferably upon informing the user (12) that the authentication was not successful.

5. The method according to one of the preceding claims, wherein before performing the authentication phase a pairing phase is performed, the pairing phase comprising the steps of
- setting the remote device (2) into pairable mode in order to be discoverable by the medical device (1),
- setting the medical device (1) into pairing mode and performing a remote device discovery inquiry by the medical device (1),
- displaying the discovered remote devices (2) for user selection,
- upon user selection of the remote device (2) pairing the medical device (1) and the remote device (2),
- upon successful pairing generating a link key on the medical device (1) and on the remote device (2),
- storing the link key and the virtual address of the remote device (2) in a non-volatile memory in the medical device (1) and storing the link key and the virtual address of the medical device (1) in a non-volatile memory in the remote device (2),
- entering connectable mode for the medical device (1), and
- connecting the remote device (2) to the medical device (1).

6. The method according to claim 5, wherein Bluetooth is used as communication protocol for the pairing phase.

7. The method according to claim 5 or 6, wherein the remote devices (2) discovered when performing the remote device discovery inquiry are filtered so that only remote devices (2) that are in the pairable mode are displayed for user selection.

8. The method according to one of the claims 5 to 7, wherein the remote devices (2) discovered when performing the remote device discovery inquiry are filtered prior to displaying for user selection by comparing the discovered remote devices (2) to entries of a predefined remote device list.

9. The method according to one of the claims 5 to 8, wherein the pairing of the medical device (1) and the remote device (2) is carried out by requesting authentication of the selected remote device (2) by the medical device (1) and/or vice versa.

10. The method according to claim 9, wherein for the authentication the medical device (1) and the selected remote device (2) each provide a fixed PIN code.
